# EUROPÄISCHE PATENTSCHRIFT

(11) **EP 2 049 540 B1**
(45) Veröffentlichungstag und Bekanntmachung des Hinweises auf die Patenterteilung: **22.12.2010**
(21) Anmeldenummer: 07764402.9
(22) Anmeldetag: 20.06.2007
(51) Int. Cl.: C07D 487/04, A61K 31/519, A61P 31/12

(54) **4-AMINO-3-ARYLAMINO-6-ARYLPYRAZOLO[3,4-D]PYRIMIDIN-DERIVATE, VERFAHREN ZU IHRER HERSTELLUNG UND DEREN VERWENDUNG ALS ANTIVIRALE WIRKSTOFFE**
4-AMINO-3-ARYLAMINO-6-ARYLPYRAZOLO[3,4-D]PYRIMIDINE DERIVATIVES, METHODS FOR THEIR PREPARATION AND THEIR USE AS ANTIVIRAL AGENTS
DÉRIVÉ DE 4-AMINO-3-ARYLAMINO-6-ARYLPYRAZOLO[3,4-D]PYRIMIDINE, SON PROCÉDÉ DE FABRICATION ET SON UTILISATION EN TANT QU'AGENTS ANTIVIRAUX

(30) Priorität: 22.06.2006 DE 102006029074
(43) Veröffentlichungstag der Anmeldung: 22.04.2009
(73) Patentinhaber: Dritte Patentportfolio Beteiligungsgesellschaft mbH & Co. KG, 12529 Schönefeld/OT Waltersdorf (DE); Makarov, Vadim, Moscow 129-090 (RU)
(72) Erfinder: MAKAROV, Vadim, Moscow 129090 (RU); WUTZLER, Peter, 99102 Erfurt-Windischholzhausen (DE); SCHMIDTKE, Michaela, 07743 Jena (DE); DAHSE, Hans-Martin, 07749 Jena (DE)
(74) Vertreter: Zech, Stefan Markus
(86) Internationale Anmeldenummer: PCT/DE2007/001104
(87) Internationale Veröffentlichungsnummer: WO 2007/147401

(56) Entgegenhaltungen:
- WO-A-00/43394

## Beschreibung

Die Erfindung betrifft neuartige 4-Amino-3-arylamino-6-arylpyrazolo[3,4-d]pyrimidin-Derivate, Verfahren zu ihrer Herstellung und deren Verwendung als antivirale Wirkstoffe, vorzugsweise zur Behandlung von Picornavirusinfektionen.

Picornaviren, insbesondere Entero- und Rhinoviren, sind für ein breites Spektrum von Erkrankungen beim Menschen verantwortlich. Zu den Enteroviren gehören mehr als 60 verschiedene humanpathogene Serotypen (Melnick J in: Fields B et al., editors. Virology. Philadelphia: Lippincott-Raven Publishers; 1996, 655-712). Enterovirus-, Echovirus-, Coxsackievirus A- und B-Infektionen verlaufen oftmals mit unspezifischem Fieber und rufen Erkrankungen des oberen Respirationstraktes hervor, die sich häufig nicht von Rhinovirusinfektionen unterscheiden lassen. Zu den schwereren Krankheitsbildern, die auch epidemisch auftreten können, gehören die hämorrhagische Konjunktivitis, Herpangina, Hand-Fuß-Mund-Krankheit, aseptische Meningitis, Enzephalitis und akute Myokarditis. Dabei können verschiedene Virustypen gleiche Symptome bzw. ein Virustyp ganz unterschiedliche Krankheitsbilder herbeiführen. Mit der Einführung moderner und sensitiver Methoden in die Virusdiagnostik gelang der Nachweis persistierender enteroviraler RNA sowie von Virusproteinen im Zusammenhang mit chronischen Erkrankungen wie z. B. dem Typ II Diabetes, Poliomyositis und vor allem der chronischen Myokarditis. Persistierende Enterovirusinfektionen kommen auch bei Patienten mit Agammaglobulinämie vor und äußern sich hierbei als persistierende Enterovirus-Meningoenzephalitis. Als Begleiterscheinungen traten häufig eine Dermatomyositis oder Polymyositis auf. Zu den Rhinoviren gehören ca. 100 Serotypen. Rhinovirusinfektionen verursachen mehr als die Hälfte aller respiratorischen Erkrankungen des oberen Respirationstraktes beim Menschen (Couch RB in: Fields BM et al., editors: Fields Virology, 3rd edition. Lippincott-Raven, Philadelphia, 1996, 713-35). Bei einer mittleren Krankheitsdauer von ca. 10 Tagen führen diese meist harmlos verlaufenden Erkältungen jährlich zu millionenfachen Arztbesuchen, Arbeits- und Schulausfällen. Als Komplikationen können eine Otitis media, Sinusitis, Exazerbation von Asthma und zystischer Fibrose sowie Infekte des unteren Respirationstraktes vor allem bei Kleinkindern, älteren Patienten und immunsupprimierten Patienten auftreten. Auf Grund der Typenvielfalt ist eine Impfprophylaxe zur Zeit nicht möglich. Bedingt durch die mit diesen Erkrankungen verbundenen Arbeitsausfälle, Arztbesuche und Medikamente verursachen Rhino- und Enteroviren jährlich enorme Kosten. Die Behandlung dieser Virusinfektionen erfolgt bisher symptombezogen, da keine virusspezifischen Therapeutika zur Verfügung stehen (Rotbart HA: Antiviral Res 2002, 53(2), 83-98). Zudem werden häufig unnütz Antibiotika verordnet. Die Entwicklung von neuen Virustatika ist deshalb unbedingt notwendig.

Die Ergebnisse der intensiven Suche nach Behandlungsmöglichkeiten von Enterovirus- und Rhinovirus-Infektionen wurden von Rotbart 2002 in einem Übersichtsartikel zusammengefasst (Rotbart HA: Antiviral Res 2002, 53(2), 83-98). Beispielsweise hemmt Ribavirin ein Wirtszellenzym, die Inosin 5'-Monophosphat (IMP)-Dehydrogenase. Durch die Ausschaltung dieses Schlüsselenzymes für die Synthese von Purinnukleotiden, lässt sich die Replikation von Picornaviren *in vitro* und *in vivo* hemmen. Ribavirin soll außerdem direkt in das Genom von Polioviren eingebaut werden und dadurch zusätzlich als Mutagen für RNA-Viren wirken (Crotty S et al.: Nat Med, 2000, 6(12),1375-9). Wegen starker Nebenwirkungen werden diese Verbindungen nicht zur Behandlung von Rhino- und Enterovirusinfektionen eingesetzt. Spezifische Targets zur Hemmung der viralen RNA-Synthese stellen das Genom an sich, die virale RNA-abhängige RNA-Polymerase sowie weitere für den Replikationskomplex notwendige virale Proteine dar. Guanidine, Thiosemicarbazone, Benzimidazole, Dipyridamole und Flavone sind seit langem als Inhibitoren der Polymerasen von verschiedenen Picornaviren in der Zellkultur bekannt. Sehr unterschiedliche Erfolge wurden damit *in vivo* erzielt. Als aussichtsreichster Kandidat mit breiter anti-Enterovirus- und anti-Rhinovirus-Aktivität gelten Enviroximderivate. Enviroxim verhindert die Synthese von Plusstrang RNA durch Bindung an das Virusprotein 3A, welches zur Bildung von RNA-Intermediaten bei der Virusvermehrung notwendig ist (Heinz BA und Vance LM: J Virol, 1995, 69(7), 4189-97). In klinischen Studien wurden moderate oder keine therapeutische Wirkungen, eine schlechte Pharmakokinetik und unerwünschte Nebenwirkungen festgestellt (Miller FD et al.: Antimicrob Agents Chemother, 1985, 27(1), 102-6). Von neueren Derivaten mit besserer Bioverfügbarkeit und Verträglichkeit liegen bisher keine klinischen Daten vor.

Basierend auf dem Wissen der Feinstruktur und Funktion der viralen Protease 2C wurde der Proteaseinhibitor AG 7088 entwickelt. AG 7088 wirkt in der Zellkultur im nanomolaren Konzentrationsbereich gegen 48 Rhinovirustypen sowie Coxsackievirus A21, B3, Enterovirus 70 und Echovirus 11 (Pattick AK et al.: Antimicrobila Agents Chemother, 1999, 43(10), 2444-50). Bisher sind die abschließenden Daten der klinischen Studien nicht bekannt.

Mit der Aufklärung der molekularen Struktur der viralen Kapside wurden die Voraussetzungen für ein zielgerichtetes Design von Kapsidblockern, den "WIN-Substanzen", geschaffen (Diana GD: Curr Med Chem 2003, 2, 1-12). Sie verhindern die Adsorption und/oder das Uncoating von Rhino- und Enteroviren. Einige der WIN-Substanzen wirken hochspezifisch nur gegen einzelne Gattungen oder Virustypen der Picornaviren. Andere Derivate hemmen die Vermehrung von Rhino- als auch Enteroviren. Zu den WIN-Substanzen gehören z. B. Arildone, Disoxaril und Pirodavir. Diese Verbindungen zeigten sehr gute antivirale Wirkungen in der Zellkultur. Eine schlechte Löslichkeit (Arildone), niedrige Bioverfügbarkeit (Arildone und Disoxaril), schnelle Metabolisierung und Ausscheidung (Disoxaril und WIN 54954) sowie Nebenwirkungen, wie beispielsweise Hautausschlag (WIN 54954), machten eine klinische Anwendung unmöglich. Große Hoffnungen wurden in Pleconaril, einen weiteren Kapsidblocker, gesetzt. Pleconaril besitzt eine sehr gute orale Bioverfügbarkeit und hemmt nach seiner Bindung in die hydrophobe Tasche im Viruskapsid die Penetration von Rhino-, Echo- und Coxsackieviren (Pevear DC et al.: Antimicrob Agents Chemother 1999, 43(9), 2109-15; McKinlay MA et al.: Annu Rev Microbiol 1992, 46, 635-54). Dadurch ist es potenziell wirksam gegen ein breites Spektrum von Viruserkrankungen, von der gewöhnlichen Erkältung bis hin zur viralen Meningitis oder Myokarditis. Resistenzen wurden bei Rhinoviren, Enterovirus 71 und Coxsackievirus B3 beobachtet (Ledford RM et al.: J Virol 2004, 78(7), 3663-74; Groarke JM et al.: J Infect Dis 1999, 179(6), 1538-41). Klinische Studien bei Kindern und Erwachsenen mit einer Enterovirusmeningitis (Abzug MJ et al.: Pediatr Infect Dis J, 2003, 22, 335-41) sowie Rhinovirus-verursachten respiratorischen Infektionen (Hayden FG et al.: Antivir Ther, 2002, 7, 53-65; Hayden FG et al.: Clin Infect Dis, 2003, 36, 1523-32) verliefen positiv. Die nachgewiesene therapeutische Wirkung reichte jedoch nicht für die Zulassung von Pleconaril (Picovir, Viropharma, USA) zur Behandlung von Rhinovirusinfektionen in den USA. Im März 2002 wurde ein entsprechender Antrag von der Nahrungs- und Arzneimittel-Behörde (Food and Drug Administration: FDA) wegen zu geringem Therapieerfolg bei gleichzeitig nachgewiesenen Nebenwirkungen abgelehnt.

Es sind auch Pyrazolopyrimidine als CRF-Antagonisten beschrieben (z. B. EP 674 642 und EP 691 128), welche beispielsweise die Adenosinkinase (EP 496 617 oder US 4,904,666), die Xanthinoxigenase (J. Heterocyc. Chem. 19, 1565, 1982) oder andere Enzymsysteme (US 2,965,643 und US 3,600,389) hemmen.

Somit besteht weiterhin als eine dringliche Aufgabe der antiviralen Forschung die Entwicklung hocheffektiver Virustatika zur Behandlung von Rhino- und Enterovirus-erkrankungen. Die neuen Verbindungen sollten gut verträglich sein und existierende Resistenzen, z. B. gegenüber Pleconaril, überwinden.

WO 00/43394 A offenbart substituierte Pyrazolo[3,4-d]pyrimidin- Derivate der allgemeinen Formel I und deren Verwendung als antivirale Wirkstoffe.

Aufgabe der Erfindung ist es, weitere Pyrazolo[3,4-d]pyrimidin- Derivate der allgemeinen Formel I sowie deren Herstellung und Verwendung anzugeben, welche als antivirale Wirkstoffe gegenüber Enteroviren und Rhinoviren einsetzbar sind sowie die angegebenen Nachteile des Standes der Technik, insbesondere die Probleme hinsichtlich Resistenz und Unverträglichkeit entsprechender Medikamente, vermeiden.

Erfindungsgemäß wird diese Aufgabe durch spezifisch substituierte 4-Amino-3-arylamino-6-arylpyrazolo[3,4-d]pyrimidin-Derivate der allgemeinen Formel I, einschließlich ihrer pharmazeutisch verträglichen Salzverbindungen, gelöst, 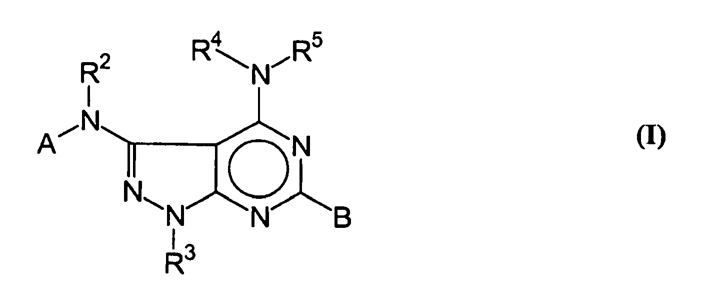 wobei:
- die Gruppen A und B von einander unabhängig Phenyl, Naphthyl, Pyridyl, Chinolyl, Pyrazinyl, Pyrimidyl, Pyrazolyl, Triazinyl, Imidazolyl, Furanyl, Thienyl sind, wobei in jeder der vorstehend genannten Gruppen unabhängig von einander ein bis drei Wasserstoffatome durch nachfolgend bezeichneten Rest R¹ ersetzt sein können,
- der Rest R¹ NO₂, CN, CONR²₂, COOR², CHO, CONH₂ ein Halogen, ein gesättigtes oder ungesättigtes, lineares oder verzweigtes aliphatisches Radikal mit 1-7 Kettengliedern, ein gesättigtes oder ungesättigtes, lineares oder verzweigtes alkanoles Radikal mit 1-8 Kettengliedern, OR², SR², NR²₂, SO₂NR³₂, Di- oder Trifluoromethyl, Phenyl sein kann,
- die Reste R², R³, R⁴, R⁵ unabhängig voneinander, H, ein gesättigtes oder ungesättigtes, halogeniertes oder nicht halogeniertes, lineares oder verzweigtes aliphatisches Radikal mit 1-7 Kettengliedern, Benzyl, Phenyl oder Naphtyl, ein gesättigter oder ungesättigter, Mono- oder Polyheterozyklus mit den Heteroatomen N, S, O ist, wobei jede der vorstehenden genannten Gruppen unabhängig mit Fluor, Chlor, Brom, Trifluormethyl, Alkyl, Alkoxy, Cyano, Nitro, Amino, Aminoalkyl, C(O)-Alkyl, C(O)O-Alkyl, Benzyl, Phenyl oder Naphtyl substituiert sein kann.

In den Unteransprüchen sind vorteilhafte Ausführungen der spezifisch substituierten 4-Amino-3-arylamino-6-arylpyrazolo[3,4-d]pyrimidin-Derivate sowie Verfahren zu ihrer Herstellung und Anwendungsmöglichkeiten ausgeführt, ohne die Erfindung jeweils darauf zu beschränken.

In einer bevorzugten Ausführung betrifft die Erfindung Verbindungen der allgemeinen Formel (I), ausgewählt aus der Gruppe der 6-Phenylaminopyrazolo[3,4-d]pyrimidine, umfassend:
4-Amino-6-phenyl-3-(tri-R¹)phenylaminopyrazolo[3,4-d]pyrimidin,
4-Amino-6-(tri-R¹)phenyl-3-phenylaminopyrazolo[3,4-d]pyrimidin,
1-Alkyl-4-amino-6-phenyl-3-(tri-R¹)phenylaminopyrazolo[3,4-d]pyrimidin,
4-Amino-1,6-di(tri-R¹)phenyl-3-phenylaminopyrazolo[3,4-d]pyrimidin,
4-Amino-6-phenyl-3-(tri-R¹)phenylalkylaminopyrazolo[3,4-d]pyrimidin,
1-Alkyl-4-amino-6-phenyl-3-(tri-R¹)phenylalkylaminopyrazolo[3,4-d]pyrimidin.

Vorteilhaft schließt die Erfindung auch 6-Phenylaminopyrazolo[3,4-d]pyrimidine der allgemeinen Formel (I) ein, umfassend:
4-Amino-3-(3-fluorophenyl)amino-6-phenylpyrazolo[3,4-d]pyrimidin,
4-Amino-3-(3-fluorophenyl)amino-6-(4-chlorophenyl)pyrazolo[3,4-d]pyrimidin,
4-Amino-3-(3-chloro)amino-6-phenylpyrazolo [3,4-d]pyrimidin,
4-Amino-3-(3-methoxy)amino-6-phenylpyrazolo [3,4-d]pyrimidin,
4-Amino-3-(4-fluorophenyl)amino-6-phenylpyrazolo [3,4-d]pyrimidin,
4-Amino-3-(4-fluorophenyl)amino-6-(4-chlorophenyl)pyrazolo [3,4-d]pyrimidin,
4-Amino-3-(4-chlorophenyl)amino-6-phenylpyrazolo[3,4-d]pyrimidin,
4-Amino-3-(3-fluorophenyl)amino-1-methyl-6-phenylpyrazolo[3,4-d]pyrimidin,
4-Amino-1-benzyl-3-(3-fluorophenyl)amino-6-phenylpyrazolo [3,4-d]pyrimidin.

Überraschenderweise zeigen die Verbindungen der vorliegenden Erfindung eine starke antivirale Aktivität gegenüber Picornaviren, insbesondere Entero- und Rhinoviren im nano- bzw. mikromolaren Konzentrationsbereich.

Daher sind die erfindungsgemäßen pharmazeutischen Zubereitungen, die eine Verbindung der Formel (I) enthalten, besonders geeignet für die Behandlung von respiratorischen Infekten, der aseptischen Meningitis, der Enzephalitis, Herpangina usw. bei Mensch und Tier, die von Picornaviren insbesondere Entero- und Rhinoviren hervorgerufen werden können.

Die Erfindung soll nachfolgend anhand von Syntheseverfahren, speziellen 4-Amino-3-arylamino-6-arylpyrazolo[3,4-d]pyrimidin-Derivaten der allgemeinen Formel (I) sowie deren Wirkung und Anwendung gegen Picornavirusinfektionen näher erläutert werden.

Abb. 1 zeigt ein allgemeines Schema zur Synthese von erfindungsgemäßem Pyrazolo[3,4-d]Pyrimidin 1 und schließt im ersten Schritt die Kondensation von [Bis(methylthio)methylen]malononitril 2 mit Arylaminen 3 in Alkohol zu Arylderivaten 4 ein. Letztere können jeweils isoliert und für weitere Reaktionen aufgereinigt werden oder direkt ohne Aufreinigung für nachfolgende Reaktion verwendet werden ("one-pot" Reaktion). Der darauf folgende Schritt besteht in der Wechselwirkung des Arylderivats 4 mit Hydrazin oder Hydrazinderivaten. Die Reaktion verläuft unter Kochen über 1 bis 4 Stunden und führt zu einer hohen Ausbeute an Pyrazol 5. Der entscheidende Schritt der Synthese von Pyrazolo[3,4-d]Pyrimidin 1 besteht in der Kondensation des Pyrazol 5 mit Arylamidinen 6 in Gegenwart von Essigsäure, Trifluoressigsäure oder Natriumacetat.

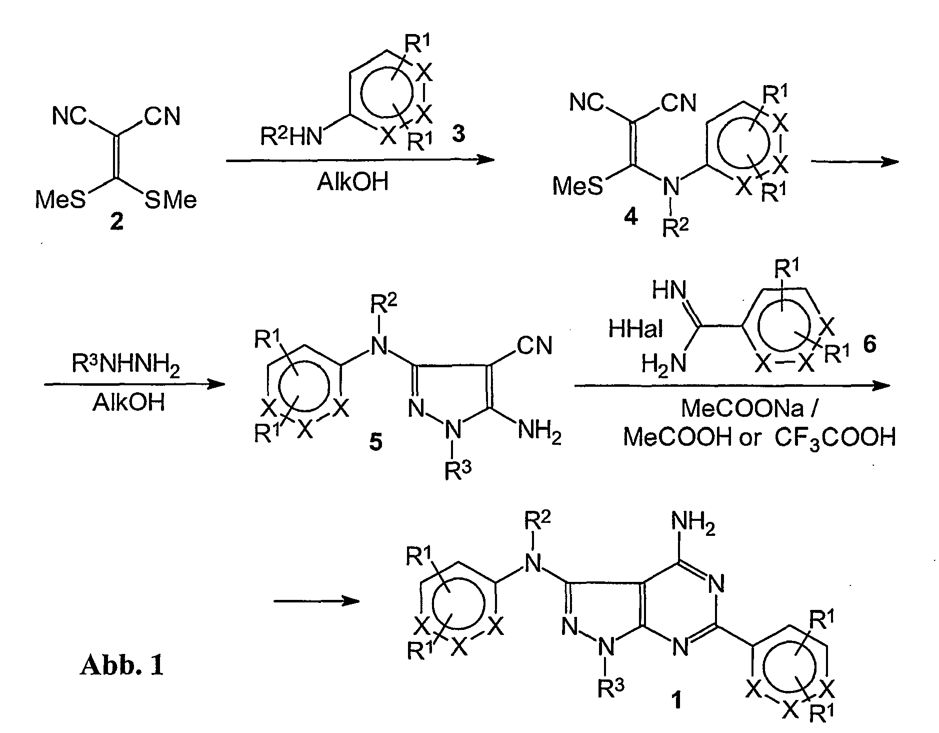

Eine alternative Synthesemethode besteht in der "one-pot" Reaktion von Malononitril mit Arylisothiocyanaten in Anwesenheit von Natriumhydrid und einer nachfolgenden Behandlung des Reaktionsgemisches mit Jodmethyl oder Dimethylsulfat. Dabei werden große Mengen an Enaminen gebildet. Auch hier ist wiederum die Kondensation von Pyrazol 5 mit Arylamidinen 6 in Gegenwart von Säure, wie Essigsäure oder Trifluoressigsäure, bzw. deren Salzen (Acetat) der entscheidende Syntheseschritt zur Herstellung von Pyrazolo[3,4-d]Pyrimidin 1.

In den nachfolgenden Beispielen sind spezielle Verbindungen der allgemeinen Formel (I) aufgelistet, welche bevorzugt für Anwendungen gegen Picornavirusinfektionen geeignet sind (ohne die Erfindung auf diese einzuschränken), wobei die erfindungsgemäßen Verbindungen in einer Lösung oder einer Suspension in einem pharmazeutisch akzeptablen wässrigen, organischen oder wässrig-organischen Medium für die lokale oder parenterale Anwendung durch intravenöse, subkutane oder intramuskuläre Injektion oder für die intranasale Verabreichung zubereitbar ist, oder sie sind in Form einer Tablette, Kapsel bzw. wässrigen Suspension mit konventionellem Träger für die orale Verabreichung oder als Suppositorium ausgebildet.

Die vorgestellten Verbindungen der Formel (I) können dabei in Dosierungen von 0,1 bis 1000 mg/kg Körpergewicht verwendet werden.

### 1. Herstellung und Analyse der 4-Amino-3-arylamino-6-arylpyrazolo[3,4-d]pyrimidin-Derivate

Die Strukturaufklärung der erfindungsgemäßen Verbindungen erfolgte durch die Art der Synthese, Elementaranalysen, NMR - und Massenspektroskopie.

Ausgangsmaterialien:

Die 5-Amino-4-cyano-3-arylaminopyrazole wurden gemäß dem in Abb. 1 dargestellten Verfahren sowie nach der Beschreibung von Tominaga Y et al. (J. Heterocycl. Chem., 1990, 27, 775-779), synthetisiert. Arylamidine werden gemäß dem bekannten Stand der Technik aus den entsprechenden Cyan-Ausgangsverbindungen synthetisiert (Boere, RT et al.: J. Organomet. Chem., 1987, 331, 161-167; Garigipati RS: Tetrahedron Lett., 1990, 31, 1969-1978; Dann O et al.: Justus Liebigs Ann. Chem., 1982, 1836-1839).

### Beispiel 1:

### 4-Amino-3-phenylamino-6-phenylpyrazolo [3,4-d] pyrimidin

Unter Rühren werden zu 2,3 g (11,5 mmol) 5-Amino-4-cyano-3-phenylaminopyrazol 3,0 g (17,24 mmol) Benzamidin Hydrochlorid Hydrat und 2,2 g (23,0 mmol) Natriumacetat zugegeben. Das Reaktionsgemisch wird für 30 min bei 220 °C erhitzt. Das resultierende Material wird mit 50 ml Wasser behandelt, gefiltert und mit 20 ml kaltem Methanol sowie 20 ml kaltem Ester gewaschen. Das Produkt wird mittels Kristallisation aus DMF/Wasser gereinigt.

Hellgelber, fester kristalliner Stoff. Ausbeute 57 %. mp 253-5 °C.
*R_{f}*(Chloroform - Methanol; 10/1) - 0.8 (Silikagel 60).
MS m/z 302 (M⁺).

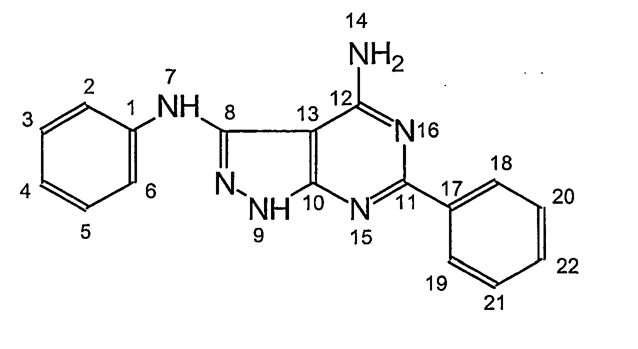

¹H NMR (DMSO-d₆) δ 12,38 (1H, s, NH(9)), 8,32-8,36 (2H, q, CH(18), CH(19)), 8,23 (1H, br. s, NH(7)), 7,67 (2H, d, CH(2), CH(6)), 7,48 (2H, br. s, NH₂), 7,42 (3H, m, CH(20), CH(21), CH(22)), 7,12 (2H, d, CH(3), CH(5)) und 6,98 (1H, m, CH(4)) ppm.
¹³C NMR (DMSO-d₆) δ 161,0 (C(11)), 156,2 (C(12)), 153,0 (C(10)), 144,2 (C(8)), 138,3 (C(17)), 136,0 (C(1)), 130,3 (C(4)), 129,8 (C(22)), 128,8 (C(3), C(5)), 128,0 und 127,7 (C(18), C(19)), 120,4 (C(4)), 120,2 (C(2), C(6)), 88,7 (C(13)) ppm.

| | | | |
|---|---|---|---|
| Berechnet für C₁₇H₁₄N₆: | C, 67,54; | H, 4,67; | N, 27,80 |
| Gefunden: | C, 67,61; | H, 4,82; | N, 27,79 |

### Beispiel 2:

### 4-Amino-3-(3-fluorophenyl)amino-6-phenylpyrazolo[3,4-d]pyrimidin

Die Herstellung erfolgte wie unter Beispiel 1 beschrieben.
Hellgelber, fester kristalliner Stoff. Ausbeute 46 %. mp 267-9 °C.
R_{f}(Chloroform - Methanol; 10/1) - 0.85 (Silikagel 60).
MS m/z 320 (M⁺).

¹H NMR (DMSO-d₆) δ 12,61 (1H, s, NH(9)), 8,35-8,38 (2H, q, CH(18), CH(19)), 8,64 (1H, br. s, NH(7)), 7,46 (2H, br. s, NH₂), 7,3-7,52 (6H, m, CH(2), CH(4), CH(6), CH(20), CH(21), CH(22)), 6,60 (1H, t, CH(5)) ppm.
¹³C NMR (DMSO-d₆) δ 166,2 und 161,2 (C(3)), 162.2 (C(11)), 161,8 (C(10)), 156,1 (C(12)), 144,3 (C(1)), 143,4 (C(8)), 130,0 (C(17)), 129,8 (C(5)), 128,5 (C(22)), 127,0 (C(18), C(19)), 112,5 (C(6)), 105,5 und 105,8 (C(2)), 102,6 und 103,9 (C(4)), 89.39 (C(13)) ppm.

| | | | |
|---|---|---|---|
| Berechnet für C17H14 F N6: | C, 63,74; | H, 4,09; | N, 26,24 |
| Gefunden: | C, 63,60; | H, 4,02; | N, 27,99 |

### Beispiel 3:

### 4-Amino-3-(3-methylphenyl)amko-6-phenylpyrazolo [3,4-d]pyrimidin

Die Herstellung erfolgte wie unter Beispiel 1 beschrieben.
Fast weißer, fester kristalliner Stoff. Ausbeute 73 %. mp 246-8 °C.
R_{f}(Chloroform - Methanol; 10/1) - 0.90 (Silikagel 60).
MS m/z 316 (M⁺).

¹H NMR (DMSO-d₆) δ 12,38 (1H, s, NH(9)), 8,32-8,36 (2H, q, CH(18), CH(19)), 8,23 (1H, br. s, NH(7)), 7,42-7,67 (6H, m, NH₂, CH(6), CH(20), CH(21), CH(22)), 7,21-7,29 (2H, m, CH(2), CH(5)) und 6,42 (1H, d, CH(4)), 2,17 (3H, s, CH₃) ppm.

| | | | |
|---|---|---|---|
| Berechnet für C₁₈H₁₆N₆: | C, 68,34; | H, 5,10; | N, 26,56 |
| Gefunden: | C, 68,43; | H, 5,16; | N, 26,71 |

### Beispiel 4:

### 4-Amino-3-(4-methylphenyl)amino-6-phenylpyrazolo[3,4-d]pyrimidin

Die Herstellung erfolgte wie unter Beispiel 1 beschrieben.
Die physiko-chemischen Parameter sind:
Fast weißer, fester kristalliner Stoff. Ausbeute 43 %. mp 266-8 °C.
R_{f}(Chloroform - Methanol; 10/1) - 0.85 (Silikagel 60).
MS m/z 316 (M⁺).

¹H NMR (DMSO-d₆) δ 12,38 (1H, s, NH(9)), 8,33-8,38 (2H, q, CH(18), CH(19)), 8,15 (1H, br. s, NH(7)), 7,60 (2H, d, CH(2), CH(6)), 7,48 (2H, br. s, NH₂), 7,42 (3H, m, CH(20), CH(21), CH(22)), 6,84 (2H, d, CH(3), CH(5)) und 2,34 (3H, s, CH₃) ppm.
¹³C NMR (DMSO-d₆) δ 161,0 (C(11)), 156,2 (C(12)), 153,0 (C(10)), 144,2 (C(8)), 138,3 (C(17)), 136,0 (C(1)), 130,3 (C(4)), 129,8 (C(22)), 128,0 und 127,7 (C(18), C(19)), 123,8 (C(3), C(5)), 118,2 (C(2), C(6)), 88,9(C(13)), 20,8(CH₃) ppm.

| | | | |
|---|---|---|---|
| Berechnet für C₁₈H₁₆N₆: | C, 68,34; | H, 5,10; | N, 26,56 |
| Gefunden: | C, 68,38; | H, 5,07; | N, 26,47 |

### Beispiel 5:

### 4-Amino-3-(4-bromophenyl) amino-6-phenylpyrazolo [3,4-d]pyrimidin

Unter Rühren werden zu einer Lösung aus 1,0 g (3,6 mmol) 5-amino-4-cyano-3-(4-bromophenyl)aminopyrazol in 20 ml Essigsäure 1,87 g (10,7 mmol) Benzamidin Hydrochlorid Hydrat und 0,89 g (10,7 mmol) Natriumacetat gegeben. Das Reaktionsgemisch wurde 4 h gekocht, mit 50 ml Wasser behandelt, filtriert und mit 20 ml kaltem Methanol sowie 20 ml kaltem Ester gewaschen. Das Rohprodukt wurde mittels Kristallisation aus Ethanol gereinigt. Die physiko-chemischen Parameter sind:
Gelber, kristalliner, fester Stoff, Ausbeute 38 %. mp 272-4 °C.
R_{f}(Chloroform - Methanol; 10/1) - 0.9 (Silikagel 60).
MS m/z 381 (M⁺).

¹H NMR (DMSO-d₆) δ 12,44 (1H, s, NH(9)), 8,33-8,38 (2H, q, CH(18), CH(19)), 8,12 (1H, br. s, NH(7)), 7,40-7,53 (7H, m, NH₂, CH(3), CH(5), CH(20), CH(21), CH(22)), 7,10 (2H, d, CH(2), CH(6)) ppm.

| | | | |
|---|---|---|---|
| Berechnet für C₁₇H₁₃BrN₆: | C, 53,65; | H, 3,44; | N, 22,04 |
| Gefunden: | C, 53,80; | H, 3,48; | N, 21,95 |

### Beispiel 6:

### 4-Amino-3-(4-fluorophenyl)amino-6-phenylpyrazolo[3,4-d]pyrimidin

Die Synthese erfolgte wie unter Beispiel 5 beschrieben, wobei als Lösungsmittel Trifluor-essigsäure eingesetzt wurde. Die Kristallisation des Endproduktes erfolgte aus Ethanol/DMF.

Die physiko-chemischen Parameter sind:
Weiß-gelber, kristalliner, fester Stoff, Ausbeute 58 %. mp 259-263 °C.
R_{f}(Chloroform - Methanol; 10/1) - 0.8 (Silikagel 60).
MS m/z 320 (M⁺).

¹H NδMR (DMSO-d₆) δ 12,69 (1H, s, NH(9)), 8,33-8,41 (4H, m, CH(2), CH(6),CH(18), CH(19)), 8,18 (1H, br. s, NH(7)), 7,58-65 (5H, m, NH₂, CH(20), CH(21), CH(22)), 7,27-7,31 (2H, m, CH(3), CH(5)) ppm.

| | | | |
|---|---|---|---|
| Berechnet für C₁₇H₁₄ F N₆: | C, 63,74; | H, 4,09; | N, 26,24 |
| Gefunden: | C, 63,57; | H, 4,07; | N, 26,33 |

### 2. Verwendung der erfindungsgemäßen 4-Amino-3-arylamino-6-arylpyrazolo[3,4-d]pyrimidin-Derivate als antivirale Wirkstoffe

### 2.1 Verträglichkeit der Verbindungen aus den vorgenannten Beispielen 1 bis 6 in der Zellkultur:

Pro Mikrotestplatten-Kavität wurden 1x10⁴ HeLa-Zellen (DSMZ, ACC 57) in 0,2 ml Kulturmedium RPMI 1640 eingesät. Die Mikrotiterplatten wurden ohne Testsubstanz standardmäßig (bei 37 °C, 5 % CO₂ und ca. 95 % relativer Luftfeuchtigkeit) 48 Stunden unter physiologischen Bedingungen inkubiert, um subkonfluente Monolayer zu erzeugen. Danach wurden die Monolayer mit Verdünnungsstufen der Testsubstanzen versetzt und 72 h lang unter physiologischen Bedingungen inkubiert. Am Ende der Inkubationszeit wurden nach Glutaraldehyd-Fixierung und Methylenblau-Färbung die Extinktion aller Kavitäten der Mikrotiterplatte bei 660 nm mit einem Mikroplatten-Reader (Sunrise, TECAN) gemessen und die CC₅₀ mit Hilfe des Auswerteprogramm "Magellan" bestimmt. Da die Vorinkubation der HeLa-Zellen bereits zur Bildung eines subkonfluenten Zellrasens führt, ist die Zytolyse bei der nachfolgenden Inkubation mit der Testsubstanz für die Bewertung ausschlaggebend.

GMK-Zellen wurden in Mikrotiterplatten ausgesät und im Brutschrank bei 5 % CO₂, 37 °C und 95 % Luftfeuchte zur Ausbildung eines Zellrasens 48 h vorinkubiert (Schmidtke M et al.: J Virol Meth, 2001, 95(1-2), 133-143). Danach wurde das Medium entfernt und die Substanzen in verschiedenen Konzentrationen (100 µl/well/Konzentration, Verdünnungsfaktor 2) in Kulturmedium aufgetragen. Für die Kontrollwertbestimmung (sechs unbehandelte Zellkontrollen) wurden jeweils 100 µl Medium eingesetzt. 72 h nach Substanzapplikation und Inkubation erfolgt die Färbung der Zellen mit Kristallviolett/Methanol. Nach dem Herauslösen des Farbstoffes wurde die optische Dichte (OD) der einzelnen Vertiefungen in einem Plattenphotometer der Firma Dynatech (550/630 nm) gemessen und mit dem Mittelwert der Zellkontrollen verglichen. Der Mittelwert der Kontrollen wurde als 100 % angenommen. Anhand der mittleren Dosis-Wirkungs-Kurven wurde mittels linearer Interpolation die 50 % zytotoxische Konzentration (CC₅₀) berechnet.

| **Beispiele** | **50 % zytotoxische Konzentration (µg/ml) in** | |
|---|---|---|
| | **HeLa-Zellen** | **GMK-Zellen** |
| **1** | 39,6 | >50 |
| **2** | 45,7 | >50 |
| **3** | 27,7 | nicht untersucht |
| **4** | >50 | >50 |
| **5** | 8,5 | 42,9 |
| **6** | 44,3 | >50 |

### 2.2 Antivirale Wirkung der Verbindungen aus den vorgenannten Beispielen 1 bis 6 in der Zellkultur:

### Zytopathischer Effekt (zpE)-Hemmtest mit dem internationalen Referenzstamm Coxsackievirus B3 Nancy (CVB3 Nancy), humanem Rhinovirus 2 und 8 (HRV2 und HRV14) in HeLa-Zellen

Die Replikation der im Test verwendeten Viren führt zur kompletten Zerstörung der Wirtszellen, einem stark ausgeprägten zytopathischen Effekt (zpE). Durch
die Zugabe antiviral wirksamer Substanzen (100 µl/well/Konzentration, Verdünnungsfaktor 2) lässt sich der Virus-induzierte zpE gezielt hemmen (Schmidtke M. et al.: J Virol Meth, 2001, 95(1-2), 133-143). Im Test wurden unbehandelte und Substanz-behandelte geschlossene Zellrasen mit einer Virusdosis infiziert, die 24 h (CVB3 Nancy) bzw. 72 h (HRV2 und HRV8) nach Infektion zu einem vollständigen zpE in den unbehandelten Viruskontrollen führt. Zu diesem Zeitpunkt wurden die noch adhärenten Zellen mit einer Kristallviolett/Formalin Lösung fixiert und gefärbt. Die Hemmung des Virusinduzierten zpE wurde nach Farbstoffelution photometrisch in einem Dynatech-Plattenreader quantifiziert. Die Berechnung der antiviralen Wirkung erfolgte durch einen Vergleich der optischen Dichten Substanz-behandelter und unbehandelter, virusinfizierter Zellen mit der durchschnittlichen optischen Dichte der Zellkontrollen, die als 100 % gesetzt wurde. Anhand der mittleren Dosis-Wirkungskurven wurde die 50 %ige Hemmkonzentration ermittelt. Als Kontrollsubstanz wurde Pleconaril eingesetzt. Die mit den Beispielsubstanzen erzielten Ergebnisse wurden in der nachfolgenden Tabelle zusammengestellt.

| **Beispiele** | **50 %ige Hemmkonzentration (µg/ml)** | | |
|---|---|---|---|
| | **CVB3 Nancy** | **HRV2** | **HRV8** |
| **Pleconaril** | unwirksam | 0,01 | 1,3 |
| **1** | 0,002 | unwirksam | unwirksam |
| **2** | 0,001 | 4,1 | 4,6 |
| **3** | 0,02 | 1,1 | 1,0 |
| **4** | 0,08 | 1,8 | 2,2 |
| **5** | 0,04 | 0,7 | 1,0 |
| **6** | 0,03 | 0,9 | 2,0 |

### Plaque-Reduktions-Test (PRT) mit der Substanz aus Beispiel 1 und Coxsackievirus B1, B2, B4, B5, B6 (CVB1, CVB2, CVB4, CVB5, CVB6)

Für die Durchführung des Tests wurden zwei bis drei Tage alte geschlossene HeLa-Zellrasen in 12-Well-Gewebekulturplatten mit 50-80 Plaque-bildenden Einheiten (PFU) infiziert (Schmidtke M et al.: J Virol Meth, 2001, 95(1-2), 133-143). Zwei nicht infizierte Vertiefungen der Platte dienten als Zellkontrolle (ZK). Nach einstündiger Virusadsorption bei 37 °C wurde der virushaltige Überstand abgesaugt. Die infizierten Zellen wurden mit einem 0,4 % Agar enthaltendem Testmedium ohne (Viruskontrollen) bzw. mit Substanz in nicht zytotoxischen Konzentrationen (Verdünnungsfaktor 2, Doppelbestimmung pro Konzentration) überschichtet und für 48 h bei 37 °C inkubiert. Nach Fixierung und Färbung der Platten mit Kristallviolett-Formalin wurde der Agar entfernt und unter fließendem Wasser gespült. Die Anzahl der virusinduzierten Plaques wurde über einer Lichtbox ausgezählt und anschließend die prozentuale Substanz-bedingte Plaquereduktion berechnet. Drei gleiche Versuchsansätze wurden durchgeführt und anhand der berechneten mittleren Dosis-Wirkungs-Kurve die Konzentration berechnet, die zu einer 50 %igen Plaquereduktion führte (IC₅₀). Die mit der Substanz aus Beispiel 1 erzielten Ergebnisse wurden in der nachfolgenden Tabelle zusammengestellt.

| **Viren** | **50 % zytotoxische Konzentration in HeLa-Zellen (CC₅₀) in µg/ml** | **50 %ige Hemmkonzentratio n in HeLa-Zellen (IC₅₀) in µg/ml** | **Selektionsindex (SI) = CC₅₀:IC₅₀** |
|---|---|---|---|
| CVB1 | 39,6 | 12,7 | 3,1 |
| CVB2 | 39,6 | 0,3 | 132 |
| CVB4 | 39,6 | 7,1 | 5,6 |
| CVB5 | 39,6 | 2,8 | 14,1 |
| CVB6 | 39,6 | 2,6 | 15,3 |

### 2.3 Akute und subakute Toxizität der Verbindungen aus den Beispielen 2 und 4 in der Maus

Die akute Toxizität der Substanzen aus den Beispielen 2 und 4 wurde in 4-5 Wochen alten Mäusen (ohne Stammbezeichnung) bestimmt. Eine 1 %ige wässrige Carboxymethylzellulose-Lösung unter Zusatz von 1-2 Tropfen TWIN-80 wurde verwendet, um eine Substanzsuspension herzustellen. Je 5 Mäusen wurden einmalig oral 1500, 2000, 2500, 3000, 4000 oder 5000 mg/kg der Substanzen aus den Beispielen 2 und 4 verabreicht. An den folgenden 3 Tagen wurde das Allgemeinbefinden der Mäuse, Gewichtsveränderungen, die rektale Temperatur und die Überlebensrate festgestellt.

Bis zur Substanzkonzentration 3000 mg/kg überlebten alle Tiere bei einmaliger Gabe der Substanzen aus den Beispielen 2 und 4 (siehe nachfolgende Tabelle). Weder das Allgemeinbefinden, noch die Rektaltemperatur oder das Körpergewicht waren beeinflusst.

Die 50 % letale Dosis der beiden Substanzen lag bei ca. 3500 mg/kg (Berechnung nach Kärber in Mayer et al. Virologische Arbeitsmethoden, Gustav-Fischer-Verlag, Jena, 1973) Nach Applikation der 5000 mg/kg-Dosierung verstarben die Tiere innerhalb von 3 bis 5 h.

| **Konzentration (mg/kg)** | **Anzahl toter/überlebender Mäuse** | |
|---|---|---|
| | **Beispiel 2** | **Beispiel 4** |
| 1500 | 0/5 | 0/5 |
| 2000 | 0/5 | 0/5 |
| 2500 | 0/5 | 0/5 |
| 3000 | 0/5 | 0/5 |
| 4000 | 3/5 | 4/5 |
| 5000 | 5/5 | 5/5 |

Auf Grundlage dieser Ergebnisse sind die Substanzen aus den Beispielen 2 und 4 bei einmaliger oraler Gabe als sehr gut verträglich einzuschätzen.

Die subakute Toxizität derselben Substanzen (Beispiele 2 und 4) wurde in vier Wochen alten Mäusen (ohne Stammbezeichnung) bestimmt. Eine 1 %ige wässrige Carboxymethylzellulose-Lösung unter Zusatz von 1-2 Tropfen TWIN-80 wurde verwendet, um Substanzsuspensionen herzustellen. Je 7 Mäusen wurden 100 mg/kg der Substanzen aus den Beispielen 2 und 4, einmal täglich über 5 Tage peroral verabreicht. Der Beobachtungszeitraum betrug 10 Tage. Eingeschätzt wurden täglich das Allgemeinbefinden, Körpergewichtsdifferenzen, Änderungen in der Rektaltemperatur und die Überlebensrate sowie zu Versuchsende nach Sektion morphologische Veränderungen der Milz, Lunge und Leber.

Die Substanzbehandlung hatte keinen Einfluss auf das Allgemeinbefinden sowie die Körpertemperatur. Das Körpergewicht nahm im Beobachtungszeitraum, wie in den unbehandelten Kontrolltieren, zu. Keines der Tiere verstarb.

Die Substanzen aus den Beispielen 2 und 4 erwiesen sich somit in einer Konzentration von 100 mg/kg bei fünfmaliger peroraler Gabe als sehr gut verträglich.

### Aufstellung der verwendeten Bezugszeichen

- 1 -: Pyrazolo[3,4-d]Pyrimidin
- 2 -: [Bis(methylthio)methylen]malononitril
- 3 -: Arylamin
- 4 -: Arylderivat
- 5 -: Pyrazol
- 6 -: Arylamidin

## Patentansprüche

1. 4-Amino-3-arylamino-6-arylpyrazolo [3,4-d]pyrimidin-Derivate, **gekennzeichnet durch** eine Verbindung der allgemeinen Formel I, 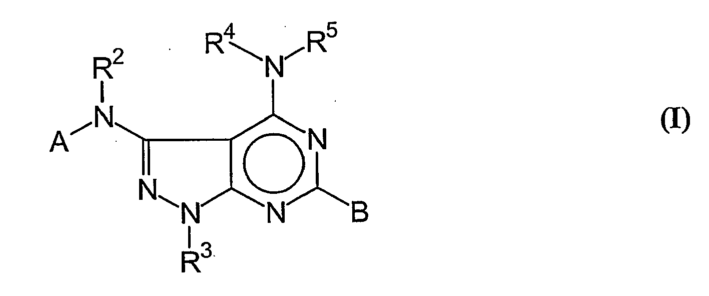 bei welcher:
- die Gruppen A und B von einander unabhängig Phenyl, Naphthyl, Pyridyl, Chinolyl, Pyrazinyl, Pyrimidyl, Pyrazolyl, Triazinyl, Imidazolyl, Furanyl, Thienyl sind, wobei in jeder der vorstehend genannten Gruppen unabhängig von einander ein bis drei Wasserstoffatome **durch** nachfolgend bezeichneten Rest R¹ ersetzt sein können,
- der Rest R¹ NO₂, CN, CONR²₂, COOR², CHO, CONH₂ ein Halogen, ein gesättigtes oder ungesättigtes, lineares oder verzweigtes aliphatisches Radikal mit 1-7 Kettengliedern, ein gesättigtes oder ungesättigtes, lineares oder verzweigtes alkanoles Radikal mit 1-8 Kettengliedern, OR², SR², NR²₂, SO₂NR³₂, Di- oder Trifluoromethyl, Phenyl sein kann,
- die Reste R², R³, R⁴, R⁵ unabhängig voneinander, H, ein gesättigtes oder ungesättigtes, halogeniertes oder nicht halogeniertes, lineares oder verzweigtes aliphatisches Radikal mit 1-7 Kettengliedern, Benzyl, Phenyl oder Naphtyl, ein gesättigter oder ungesättigter, Mono- oder Polyheterozyklus mit den Heteroatomen N, S, O ist, wobei jede der vorstehenden genannten Gruppen unabhängig mit Fluor, Chlor, Brom, Trifluormethyl, Alkyl, Alkoxy, Cyano, Nitro, Amino, Aminoalkyl, C(O)-Alkyl, C(O)O-Alkyl, Benzyl, Phenyl oder Naphtyl substituiert sein kann.

2. 4-Amino-3-arylamino-6-arylpyrazolo[3,4-d]pyrimidin-Derivate gemäß Anspruch 1, **gekennzeichnet durch** ein 4-Amino-6-phenyl-3-(tri-R¹)phenylaminopyrazolo[3,4-d]pyrimidin der allgemeinen Verbindung I, wobei der Rest R¹ in den Gruppen A und B unabhängig voneinander **durch** CONH₂, CN, Halogen, NO₂ bzw. CF₃ gebildet wird.

3. 4-Amino-3-arylamino-6-arylpyrazolo[3,4-d]pyrimidin-Derivate gemäß Anspruch 1, **gekennzeichnet durch** ein 1-R³-4-amino-6-phenyl-3-(tri-R¹)phenylaminopyrazolo[3,4-d]pyrimidin der allgemeinen Verbindung I, wobei der Rest R¹ in den Gruppen A und B unabhängig voneinander **durch** CONH₂, CN, Halogen, NO₂, CF₃ gebildet wird.

4. 4-Amino-3-arylamino-6-arylpyrazolo[3,4-d]pyrimidin-Derivate gemäß Anspruch 1, **dadurch gekennzeichnet, dass** diese aus der Gruppe der 6-Phenylaminopyrazolo[3,4-d]pyrimidine ausgewählt sind, umfassend:
4-Amino-6-phenyl-3-(tri-R¹)phenylaminopyrazolo[3,4-d]pyrimidin,
4-Amino-6-(tri-R¹)phenyl-3-phenylaminopyrazolo[3,4-d]pyrimidin,
1-Alkyl-4-amino-6-phenyl-3-(tri-R¹)phenylaminopyrazolo[3,4-d]pyrimidin,
4-Amino-1,6-di(tri-R¹)phenyl-3-phenylaminopyrazolo[3,4-d]pyrimidin,
4-Amino-6-phenyl-3-(tri-R¹)phenylalkylaminopyrazolo[3,4-d]pyrimidin,
1-Alkyl-4-amino-6-phenyl-3-(tri-R¹)phenylalkylaminopyrazolo[3,4-d]pyrimidin.

5. 4-Amino-3-arylamino-6-arylpyrazolo[3,4-d]pyrimidin-Derivate gemäß Anspruch 1, **dadurch gekennzeichnet, dass** diese aus der Gruppe der 6-Phenylaminopyrazolo[3,4-d]pyrimidine ausgewählt sind, wobei der Rest R¹ in den Gruppen A und B unabhängig voneinander durch ein Halogen gebildet wird.

6. 4-Amino-3-arylamino-6-arylpyrazolo[3,4-d]pyrimidin-Derivate gemäß Anspruch 1, **dadurch gekennzeichnet, dass** diese aus der Gruppe der 6-Phenylaminopyrazolo[3,4-d]pyrimidine ausgewählt sind, umfassend:
4-Amino-3-(3-fluorophenyl)amino-6-phenylpyrazolo[3,4-d]pyrimidin,
4-Amino-3-(3-fluorophenyl)amino-6-(4-chlorophenyl)-pyrazolo[3,4-d]pyrimidin,
4-Amino-3-(3-chloro)amino-6-phenylpyrazolo[3,4-d]pyrimidin,
4-Amino-3-(3-methoxy)amino-6-phenylpyrazolo[3,4-d]pyrimidin,
4-Amino-3-(4-fluorophenyl)amino-6-phenylpyrazolo [3,4-d]pyrimidin,
4-Amino-3-(4-fluorophenyl)amino-6-(4-chlorophenyl)pyrazolo[3,4-d]pyrimidin,
4-Amino-3-(4-chlorophenyl)amino-6-phenylpyrazolo[3,4-d]pyrimidin,
4-Amino-3-(3-fluorophenyl)amino-1-methyl-6-phenylpyrazolo [3,4-d]pyrimidin,
4-Amino-1-benzyl-3-(3-fluorophenyl)amino-6-phenylpyrazolo[3,4-d]pyrimidin.

7. Verfahren zur Herstellung von 4-Amino-3-arylamino-6-arylpyrazolo[3,4-d]pyrimidin-Derivaten gemäß Anspruch 1, **gekennzeichnet durch** Kondensation von Pyrazol mit Arylamidinen in Gegenwart von Säure (Essigsäure, Trifluoressigsäure) oder deren Salzen (Acetat).

8. Verwendung von 4-Amino-3-arylamino-6-arylpyrazolo[3,4-d]pyrimidinDerivaten gemäß einem der Ansprüche 1 bis 6 oder ihren pharmazeutisch verträglichen Salzen zur Herstellung eines Arzneimittels mit biologischer Wirkung.

9. Verwendung gemäß Anspruch 8, **dadurch gekennzeichnet, dass** die biologische Wirkung eine antivirale ist.

10. Verwendung gemäß Anspruch 9, **dadurch gekennzeichnet, dass** das Arzneimittel zur prophylaktischen und therapeutischen Anwendung gegen Virusinfektionen einsetzbar ist.

11. Verwendung gemäß Anspruch 10, **dadurch gekennzeichnet, dass** die Virusinfektionen Picornavirusinfektionen sind.

12. Verwendung gemäß einem der Ansprüche 8 bis 11, **dadurch gekennzeichnet, dass** die 4-Amino-3-arylamino-6-arylpyrazolo[3,4-d]pyrimidin-Derivate als Einzelsubstanz eingesetzt werden.

13. Verwendung gemäß einem der Ansprüche 8 bis 11, **dadurch gekennzeichnet, dass** die 4-Amino-3-arylamino-6-arylpyrazolo[3,4-d]pyrimidin-Derivate zusammen mit anderen Wirkstoffen, insbesondere mit bekannten Arzneimitteln, eingesetzt werden.

## Claims

1. 4-amino-3-arylamino-6-arylpyrazolo[3,4-d]pyrimidine derivatives, **characterized by** a compound of the general formula I, 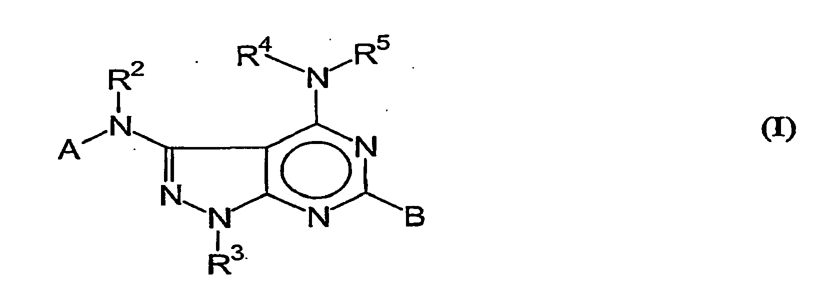 wherein:
- the groups A and B are, independently of each other, phenyl, naphthyl, pyridyl, quinoyl, pyrazinyl, pyrimidyl, pyrazolyl, triazinyl, imidazolyl, furanyl, thienyl, wherein in each of the aforementioned groups, one to three hydrogen atoms may be substituted independently of each other by the residue R¹ described below,
- the residue R¹ may be NO₂, CN, CONR²₂, COOR², CHO, CONH₂, a halogen, a saturated or unsaturated, linear or branched aliphatic radical with 1-7 chain links, a saturated or unsaturated, linear or branched alkanol radical with 1-8 chain links, OR², SR², NR²₂, SO₂NR³₂, difluoromethyl or trifluoromethyl, phenyl,
- the residues R², R³, R⁴, R⁵ are independently of each other H, a saturated or unsaturated, halogenated or non-halogenated, linear or branched aliphatic radical with 1-7 chain links, benzyl, phenyl or naphthyl, a saturated or unsaturated monoheterocyle or polyheterocycle with the heteroatoms N, S, O, wherein each of the aforementioned groups can be substituted independently of each other with fluorine, chlorine, bromine, trifluoromethyl, alkyl, alkoxy, cyano, nitro, amino, aminoalkyl, C(O)-alkyl, C(O)O-alkyl, benzyl, phenyl or naphthyl.

2. 4-amino-3-arylamino-6-arylpyrazolo[3,4-d]pyrimidine derivatives according to claim 1, **characterized by** a 4-amino-6-phenyl-3-(tri-R¹)phenylaminopyrazolo[3,4-d]pyrimidine of the general compound I, wherein the residue R¹ in the groups A and B is, independently of each other, formed by CONH₂, CN, halogen, NO₂ or CF₃.

3. 4-amino-3-arylamino-6-arylpyrazolo[3,4-d]pyrimidine derivatives according to claim 1, **characterized by** a 1-R³-4-amino-6-phenyl-3-(tri-R¹)phenylaminopyrazolo[3,4-d]pyrimidine of the general compound I, wherein the residue R¹ in the groups A and B is formed, independently of each other, by CONH₂, CN, halogen, NO₂, CF₃.

4. 4-amino-3-arylamino-6-arylpyrazolo[3,4-d]pyrimidine derivatives according to claim 1, **characterized in that** same are selected from the group of 6-phenylaminopyrazolo[3,4-d]pyrimidines, comprising:
4-amino-6-phenyl-3-(tri-R¹)phenylaminopyrazolo[3,4-d]pyrimidine,
4-amino-6-(tri-R¹)phenyl-3-phenylaminopyrazolo[3,4-d]pyrimidine,
1-alkyl-4-amino-6-phenyl-3-(tri-R¹)phenylaminopyrazolo[3,4-d]pyrimidine,
4-amino-1,6-di(tri-R¹)phenyl-3-phenylaminopyrazolo[3,4-d]pyrimidine,
4-amino-6-phenyl-3-(tri-R¹)phenylalkylaminopyrazolo[3,4-d]pyrimidine,
1-alkyl-4-amino-6-phenyl-3-(tri-R¹)phenylalkylaminopyrazolo[3,4-d]pyrimidine.

5. 4-amino-3-arylamino-6-arylpyrazolo[3,4-d]pyrimidine derivatives according to claim 1, **characterized in that** same are selected from the group of 6-phenylaminopyrazolo[3,4-d]pyrimidines, wherein the residue R¹ in the groups A and B is formed, independently of each other, by a halogen.

6. 4-amino-3-arylamino-6-arylpyrazolo[3,4-d]pyrimidine derivatives according to claim 1, **characterized in that** same are selected from the group of 6-phenylaminopyrazolo[3,4-d]pyrimidines, comprising:
4-amino-3-(3-fluorophenyl)amino-6-phenylpyrazolo[3,4-d]pyrimidine,
4-amino-3-(3-fluorophenyl)amino-6-(4-chlorophenyl)-pyrazolo[3,4-d]pyrimidine,
4-amino-3-(3-chloro)amino-6-phenylpyrazolo[3,4-d]pyrimidine,
4-amino-3-(3-methoxy)amino-6-phenylpyrazolo[3,4-d]pyrimidine,
4-amino-3-(4-fluorophenyl)amino-6-phenylpyrazolo[3,4-d]pyrimidine,
4-amino-3-(4-fluorophenyl)amino-6-(4-chlorophenyl)pyrazolo[3,4-d]pyrimidine,
4-amino-3-(4-chlorophenyl)amino-6-phenylpyrazolo[3,4-d]pyrimidine,
4-amino-3-(3-fluorophenyl)amino-1-methyl-6-phenylpyrazolo[3,4-d]pyrimidine,
4-amino-1-benzyl-3-(3-fluorophenyl)amino-6-phenylpyrazolo[3,4-d]pyrimidine.

7. A method for producing 4-amino-3-arylamino-6-arylpyrazolo[3,4-d]pyrimidine derivatives according to claim 1, **characterized by** condensing pyrazol with aryl amidines in the presence of acid (acetic acid, trifluoroacetic acid) or the salts thereof (acetate).

8. Use of 4-amino-3-arylamino-6-arylpyrazolo[3,4-d]pyrimidine derivatives according to any one of claims 1 to 6 or the pharmaceutically acceptable salts thereof for producing a pharmaceutical having biological activity.

9. Use according to claim 8, **characterized in that** the biological activity is an antiviral activity.

10. Use according to claim 9, **characterized in that** the pharmaceutical can be used for prophylactic and therapeutic administration against viral infections.

11. Use according to claim 10, **characterized in that** the viral infections are picornavirus infections.

12. Use according to any one of claims 8 to 11, **characterized in that** the 4-amino-3-arylamino-6-arylpyrazolo[3,4-d]pyrimidine derivatives are used as a single substance.

13. Use according to any one of claims 8 to 11, **characterized in that** the 4-amino-3-arylamino-6-arylpyrazolo[3,4-d]pyrimidine derivatives are used in combination with other active substances, in particular with known pharmaceuticals.

## Revendications

1. Dérivés de 4-amino-3-arylamino-6-arylpyrazolo[3,4-d]pyrimidine, **caractérisés par** une liaison de formule générale I, 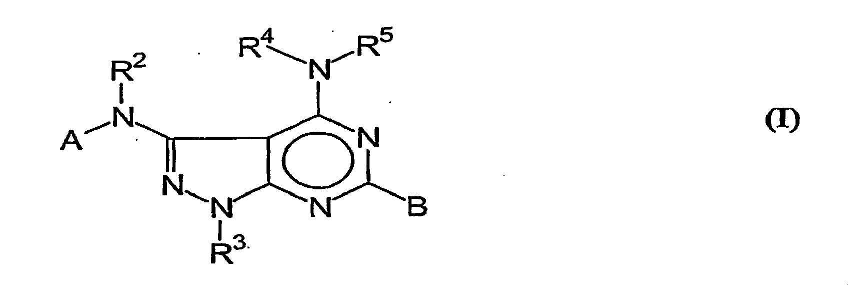 dans laquelle :
- les groupes A et B sont, indépendamment l'un de l'autre, du phényle, naphtyle, pyridyle, chinolyle, pyrazinyle, pyrimidyle, pyrazolyle, triazinyle, imidazolyle, furanyle, thienyle, un à trois atomes d'hydrogène pouvant être remplacés, dans chacun des groupes précités, indépendamment l'un de l'autre, par un reste R¹ désigné ci-après,
- le reste R¹ pouvant être NO₂, CN, CONR²₂, COOR², CHO, CONH₂, un halogène, un radical aliphatique saturé ou insaturé, linéaire ou ramifié, ayant 1 à 7 éléments de chaîne, un radical alkanole saturé ou insaturé, linéaire ou ramifié, ayant 1 à 8 éléments de chaîne, OR², SR², NR²₂, SO₂NR³₂, du di- ou trifluorométhyle, du phényle,
- les restes R², R³, R⁴, R⁵ étant, indépendamment les uns des autres, H, un radical aliphatique saturé ou insaturé, halogéné ou non halogéné, linéaire ou ramifié, ayant 1 à 7 éléments de chaîne, du benzyle, phényle ou naphtyle, un cycle mono ou polyhétéro saturé ou insaturé renfermant les atomes hétéros N, S, O, chacun des groupes précités pouvant être substitués indépendamment les uns des autres par du fluor, chlore, brome, trifluorméthyle, alkyle, alkoxy, cyano, nitro, amino, aminoalkyle, C(O)-alkyle, C(O)O-alkyle, benzyle, phényle ou naphtyle.

2. Dérivés de 4-amino-3-arylamino-6-arylpyrazolo[3,4-d]pyrimidine selon la revendication 1, **caractérisés par** une 4-amino-6-phényle-3-(tri-R¹)phénylaminopyrazolo[3,4-d]pyrimidine de liaison générale I, le reste R¹ étant constitué dans les groupes A et B, indépendamment l'un de l'autre, de CONH₂, CN, halogène, NO₂ ou CF₃.

3. Dérivés de 4-amino-3-arylamino-6-arylpyrazolo[3,4-d]pyrimidine selon la revendication 1, **caractérisés par** une 1-R³-4-amino-6-phényle-3-(tri-R¹)phénylaminopyrazolo[3,4-d]pyrimidine de liaison générale I, le reste R¹ étant constitué dans les groupes A et B, indépendamment l'un de l'autre, de CONH₂, CN, halogène, NO₂, CF₃.

4. Dérivés de 4-amino-3-arylamino-6-arylpyrazolo[3,4-d]pyrimidine selon la revendication 1, **caractérisés en ce que** ceux-ci sont sélectionnés dans le groupe des 6-phénylaminopyrazolo[3,4-d]pyrimidines, comportant :
4-amino-6-phényle-3-(tri-R¹)phénylaminopyrazolo[3,4-d]pyrimidine,
4-amino-6-(tri-R¹)phényle-3-phénylaminopyrazolo[3,4-d]pyrimidine,
1-alkyle-4-amino-6-phényle-3-(tri-R¹)phénylaminopyrazolo[3,4-d]pyrimidine,
4-amino-1,6-di(tri-R¹)phényle-3-phénylaminopyrazolo[3,4-d]pyrimidine,
4-amino-6-phényle-3-(tri-R¹)phénylalkylaminopyrazolo[3,4-d]pyrimidine, 1-alkyle-4-amino-6-phényle-3-(tri-R¹)phénylalkylaminopyrazolo[3,4-d]pyrimidine.

5. Dérivés de 4-amino-3-arylamino-6-arylpyrazolo[3,4-d] pyrimidine selon la revendication 1, **caractérisés en ce que** ceux-ci sont sélectionnés dans le groupe des 6-phénylaminopyrazolo[3,4-d] pyrimidines, le reste R¹ étant constitué dans les groupes A et B, indépendamment l'un de l'autre, d'un halogène.

6. Dérivés de 4-amino-3-arylamino-6-arylpyrazolo[3,4-d]pyrimidine selon la revendication 1, **caractérisés en ce que** ceux-ci sont sélectionnés dans le groupe des 6-phénylaminopyrazolo[3,4-d]pyrimidines, comportant :
4-amino-3-(3-fluorophényle)amino-6-phénylpyrazolo[3,4-d]pyrimidine,
4-amino-3-(3-fluorophényle)amino-6-(4-chlorophényle)-pyrazolo[3,4-d]pyrimidine,
4-amino-3-(3-chloro)amino-6-phénylpyrazolo[3,4-d]pyrimidine,
4-amino-3-(3-méthoxy)amino-6-phénylpyrazolo[3,4-d]pyrimidine,
4-amino-3-(4-fluorophényle)amino-6-phénylpyrazolo[3,4-d]pyrimidine,
4-amino-3-(4-fluorophényle)amino-6-(4-chlorophényle)pyrazolo[3,4-d]pyrimidine,
4-amino-3-(4-chlorophényle)amino-6-phénylpyrazolo[3,4-d]pyrimidine,
4-amino-3-(3-fluorophényle)amino-1-méthyle-6-phénylpyrazolo[3,4-d]pyrimidine,
4-amino-1-benzyle-3-(3-fluorophényle)amino-6-phénylpyrazolo[3,4-d]pyrimidine.

7. Procédé de production de dérivés de 4-amino-3-arylamino-6-arylpyrazolo[3,4-d]pyrimidine selon la revendication 1, **caractérisé par** la condensation de pyrazole avec des arylamidines en présence d'acide (acide acétique, acide trifluoracétique) ou de leurs sels (acétate).

8. Utilisation de dérivés de 4-amino-3-arylamino-6-arylpyrazolo[3,4-d]pyrimidine selon l'une des revendications 1 à 6 ou de leurs sels pharmaceutiquement acceptables afin de produire un médicament à action biologique.

9. Utilisation selon la revendication 8, **caractérisée en ce que** l'action biologique est une action antivirale.

10. Utilisation selon la revendication 9, **caractérisée en ce que** le médicament peut être employé à usage prophylactique et thérapeutique contre les infections virales.

11. Utilisation selon la revendication 10, **caractérisée en ce que** les infections virales sont des infections picornavirales.

12. Utilisation selon l'une des revendications 8 à 11, **caractérisée en ce que** les dérivés de 4-amino-3-arylamino-6-arylpyrazolo[3,4-d]pyrimidine sont employés comme substance individuelle.

13. Utilisation selon l'une des revendications 8 à 11, **caractérisée en ce que** les dérivés de 4-amino-3-arylamino-6-arylpyrazolo[3,4-d]pyrimidine sont employés en combinaison avec d'autres substances actives, en particulier avec des médicaments connus.
